Europäisches Patentamt

European Patent Office

Office européen des brevets

(19)

(11) Veröffentlichungsnummer : **0 391 172 B1**

(12) # EUROPÄISCHE PATENTSCHRIFT

(45) Veröffentlichungstag der Patentschrift :
05.05.93 Patentblatt 93/18

(51) Int. Cl.$^5$ : **A61M 35/00, A61L 15/16**

(21) Anmeldenummer : **90105527.7**

(22) Anmeldetag : **23.03.90**

(54) **Transdermales therapeutisches System mit erhöhtem Wirkstofffluss und Verfahren zu seiner Herstellung.**

(30) Priorität : **01.04.89 DE 3910543**

(43) Veröffentlichungstag der Anmeldung :
**10.10.90 Patentblatt 90/41**

(45) Bekanntmachung des Hinweises auf die
Patenterteilung :
**05.05.93 Patentblatt 93/18**

(84) Benannte Vertragsstaaten :
**AT BE CH DE DK ES FR GB GR IT LI LU NL SE**

(56) Entgegenhaltungen :
**EP-A- 0 040 861**
**EP-A- 0 196 769**
**GB-A- 1 361 289**
**US-A- 3 996 934**

(56) Entgegenhaltungen :
**DRUG DEVELOPMENT AND INDUSTRIAL**
**PHARMACY, Band 13(4 & 5), 1987, Seiten**
**589-651, Marcel Dekker, Inc.; Y.W. CHIEN:**
**"Development of transdermal drug delivery**
**systems"**

(73) Patentinhaber : **LTS Lohmann**
**Therapie-Systeme GmbH & Co. KG**
**Irlicherstrasse 55**
**W-5450 Neuwied 12 (DE)**

(72) Erfinder : **Horstmann, Michael, Dr.**
**Deichstrasse 9**
**W-5450 Neuwied 1 (DE)**
Erfinder : **Herrmann, Fritz, Dr.**
**Rheinheldestrasse 12c**
**W-5450 Neuwied 12 (DE)**

(74) Vertreter : **Flaccus, Rolf-Dieter, Dr.**
**Patentanwalt Sperlingsweg 32**
**W-5047 Wesseling (DE)**

EP 0 391 172 B1

**Beschreibung**

Die Erfindung betrifft ein transdermales therapeutisches System mit geschichtetem Aufbau, bestehend aus einer im wesentlichen wirkstoffundurchlässigen Rückschicht, einer den Wirkstoff enthaltenden aktivierbaren Matrix und einer den Hautfeuchtigkeitszutritt kontrollierenden Schicht, sowie ein Verfahren zur Herstellung des Systems.

Transdermale therapeutische Systeme sind auf der Haut aufzubringende, selbstklebende galenische Zübereitungen mit festgelegter Applikationsfläche, die einen Arzneistoff nach Zeit und Menge kontrolliert an den menschlichen oder tierischen Körper abgeben. Derartige Systeme, die beispielsweise von Y.W.Chien, Drug Dev. Ind.Pharm. 13, 589-651 (1987), beschrieben sind, haben sich seit Jahren in der Therapie bewährt.

Übliche Bau formen von transdermalen Systemen, die bereits Eingang in die Praxis gefunden haben, sind:

a) Aufbau aus undurchlässigem Träger und einer zweiten, gleichzeitig als Arzneistoffreservoir, Haftkleber und Steuereinheit dienenden Schicht,

b) Aufbau aus Träger, Arzneistoffreservoir, Kontrolleinheit und Klebschicht in räumlicher Trennung,

c) Aufbau aus Träger und mehrschichtig angeordneter arzneistoffhaltiger Matrix, wobei die Wirkstoffkonzentration von Schicht zu Schicht zur Haut hin geringer wird,

d) Aufbau aus Träger und Matrix, wobei die Freisetzung durch in der Matrix dispergierte arzneistoffhaltige Mikrokapseln kontrolliert wird.

Der therapeutische Fortschritt dieser Systeme gegenüber traditionellen Applikationsformen besteht darin, daß der Wirkstoff dem Körper nicht stoßweise zugeführt wird, wie beispielsweise bei Einnahme von Tabletten, sondern kontinuierlich.

Dadurch wird einerseits die Wirkungsdauer eines Arzneistoffes verlängert, zum anderen werden Nebenwirkungen durch Vermeidung unnötiger Blutspiegelspitzen weitgehend verhindert.

Da die menschliche Haut jedoch nicht für alle in Betracht kommenden Arzneistoffe eine ausreichende Durchlässigkeit aufweist, ist in transdermalen therapeutischen Systemen herkömmlicher Bauart lediglich eine geringe Anzahl von Wirkstoffen einsetzbar. Es sind daher zahlreiche Versuche mit dem Ziel unternommen worden, die natürliche Durchlässigkeit der Haut zu steigern.

Eine solche Möglichkeit ist die Verwendung von sogenannten "Penetrationsverstärkern". Man versteht hierunter Stoffe, die durch chemisch-physikalische Wechselwirkung mit der Mikrostruktur der Haut eine deutliche Steigerung des Wirkstoffflußes erzielen. Viele dieser Stoffe wirken jedoch auf der Haut toxisch oder verursachen Irritationen. Auch setzt die Wirkung dieser Resorptionsförderer nicht immer schnell genug ein, so daß der Effekt schwer zu steuern ist.

Eine andere Möglichkeit ist die Anwendung physikalischer Prinzipien, wie beispielsweise der Iontophorese. Diese Verfahren erfordern jedoch vergleichsweise aufwendige Zusatzeinrichtungen im transdermalen therapeutischen System, welche in aller Regel diese Form der Therapie unwirtschaftlich machen.

Ein grundsätzlich anderer Weg, die Hautdurchlässigkeit zu erhöhen, liegt in der Erhöhung der thermodynamischen Aktivität des Wirkstoffes. Diesbezügliche Versuche zielten auf eine Erhöhung der von außen wirkenden Konzentration des Wirkstoffs ab, um die Permeation zu steigern. Diese Bemühungen fanden ihre Grenze darin, daß im allgemeinen die Konzentration eines Wirkstoffs nicht über die Sättigungslöslichkeit hinaus gesteigert werden kann, zum anderen führt es auch nicht weiter, im transdermalen therapeutischen System galenische Grundlagen mit höherer Löslichkeit für den Wirkstoff zu benutzen, da hier die Verknüpfung zwischen Verteilungskoeffizient und Löslichkeit gemäß dem Nernstschen Verteilungsgesetz zum Tragen kommt.

Vorübergehend können sogenannte übersättigte Zustände entstehen, bei denen die gelöste Wirkstoffkonzentration über der Sättigungskonzentration liegt, z.B. beim Abkühlen einer gesättigten Lösung oder beim Wiederauflösen einer Wirkstoffeinbettung in einem wasserlöslichen Polymer, wie beispielsweise bei Merkle, Pharm. Ind. 42, 1009-1018 (1980), beschrieben ist.

Je nach dem Grad der Übersättigung und der Viskosität des umgebenden Mediums kann dieser Zustand von wenigen Sekunden bis zu mehreren Jahren andauern. In klebenden Polymermatrices mit ihrer immer noch recht geringen Viskosität bleibt er im allgemeinen über höchstens wenigen Woche stabil. Die Dauer des stabilen Zustandes wird unter anderem durch die Möglichkeit beeinträchtigt, daß die vorhandenen Grenzflächen als Kristallisationskeime wirken können.

Die Herstellung von lagerfähigen transdermalen therapeutischen Systemen mit übersättigter Lösung des enthaltenden Wirkstoffes stößt daher auf größte Schwierigkeiten.

In der EP-A-0 186 019 wird beschrieben, daß durch Zugabe wasserquellbarer Polymere zu einer Lösung des Wirkstoffs in einer Polymergrundmasse die Geschwindigkeit der Freisetzung aus einem aus dieser Masse hergestellten transdermalen therapeutischen Systems deutlich gesteigert wird.

Bei dem dort beschriebenen Verfahren wird die Zubereitung der in lipophilen Lösungsmitteln angesetzten Grundmasse jedoch in einem Arbeitsgang mit Wirkstoff und wasserquellbarem Zusatzstoff versetzt, so daß eine gezielte Anreicherung des Wirkstoffs in den hydrophilen Domänen nicht möglich ist. Der Zusatz von Hautfeuchtigkeit erfolgt unkontrolliert.

Wird der übersättigte Zustand erst erzeugt, während sich die Arzneiform bereits auf der Haut befindet, so treten naturgemäß Lagerstabilitätsprobleme nicht auf. Coldman et al (J. Pharm. Sci. 58, 1098-1102 (1969) beschreiben eine Resorptionsverstärkung von Fluocinolon durch Lösen in einem Gemisch aus flüchtigen und nichtflüchtigen Lösungsmitteln, wobei bei Verdunsten der leichter flüchtigen Komponente eine übersättigte Lösung entsteht, die zu einer gesteigerten Hautpenetration führt. Diese Effekte wurden von Kondo et al. (J. Pharmacobio.Dyn. 10, 662-668 (1987)) an Nifedipin bestätigt.

Der vorliegenden Erfindung liegt somit die Aufgabe zugrunde, ein transdermales therapeutisches System mit erhöhtem Wirkstofffluß und lagerstabiler Formulierung sowie ein Verfahren zu seiner Herstellung bereitzustellen.

Diese Aufgabe wurde gelöst durch die Bereitstellung eines transdermalen therapeutischen Systems mit schichtweisem Aufbau aus einer im wesentlichen wirkstoffundurchlässigen Rückschicht (11), einer den Wirkstoff enthaltenden aktivierbaren Matrix (12) und einer den Zutritt von Hautfeuchtigkeit kontrollierenden Schicht (13), welches dadurch gekennzeichnet ist, daß die Matrix aus einem wasserdampfdurchlässigen, jedoch im wesentlichen wasserunlöslichen und weitgehend wirkstofffreien Grundmaterial (15) besteht, in dem aus einer festen Lösung eines Arzneiwirkstoffes in einem wasserlöslichen oder in Wasser quellbaren Basismaterial bestehende Inseln (14) verteilt sind, daß die Matrix durch Hautfeuchtigkeit aktivierbar ist, wobei ein kontrollierter Zustrom von Hautfeuchtigkeit in die Matrix (12) erfolgt, und daß durch Feuchtigkeitsaufnahme der Inseln (14) eine systemkontrollierte, gezielte Übersättigung mit Wirkstoff stattfindet, die zu einer gesteigerten Arzneistoffabgabe führt, sowie durch ein Verfahren zur Herstellung des therapeutischen Systems nach den Merkmalen der Ansprüche 12 bis 19.

Geeignete Wirkstoffe finden sich in den Wirkstoffgruppen der Parasympatholytika (z.B. Scopolamin, Atropin, Benactyzin), der Cholinergika (z.B. Physostigmin, Nicotin), der Neuroleptika (z.B. Chlorpromazin, Haloperidol), der Monoaminoxidasehemmer (z.B. Tranylcypromin, Selegilin), der Sympathomimetika (z.B. Ephedrin, D-Norpseudoephedrin, Salbutamol, Fenfluramin), der Sympatholytika und Antisympathotonika (z.B. Propanolol, Timolol, Bupranolol, Clonidin, Dihydroergotamin, Naphazolin), der Anxiolytika (z.B. Diazepam, Triazolam), der Lokalanesthetika (z.B. Lidocain), der zentralen Analgetika (z.B. Fentanyl, Sufentanil), der Antirheumatika (z.B. Indomethacin, Piroxicam, Lornoxicam), der Koronartherapeutika (z.B. Glyceroltrinitrat, Isosorbiddinitrat), der Östrogene, Gestagene und Androgene, der Antihistaminika (z.B Diphenhydramin, Clemastin, Terfenadin), der Prostaglandinderivate, der Vitamine (z.B. Vitamin E, Cholecalciferol) und der Cytostatika. Darüber hinaus eignen sich weitere Wirkstoffe für den erfindungsgemäßen Zweck, wenn sie eine therapeutische Tagesdosis von weniger als 50 mg besitzen und sowohl in Wasser als auch in organischen Lösungsmitteln löslich sind.

Als Bestandteile im Grundmaterial (15, 25) der Matrix (12, 22) können dabei Polymere wie Polyisobutylen, Ester des Polyvinylalkohols, Polyacryl- und Polymethacrylsäureester, Naturkautschuk, Styrol-, Isopren- und Styrol-Butadien-Polymerisate oder Siliconpolymere, Harzbestandteile wie gesättigte und ungesättigte Kohlenwasserstoffharze, Derivate des Abietylalkohols und des ß-Pinens, Weichmacher wie Phthalsäureester, Triglyceride und Fettsäuren, sowie eine Reihe weiterer, dem Fachmann bekannter Stoffe Verwendung finden.

Für Komponenten des Basismaterials der Inseln (14,24) kommt eine ganze Reihe in Wasser quellbarer pharmazeutischer Hilfsstoffe, wie zum Beispiel Polyvinylpyrrolidon, Polyacrylsäure, Polyvinylalkohol, Cellulose und ihre Derivate, natürlich vorkommende Schleimbildner wie Agar-Agar, Guar-Gummi und Gummi arabicum, aber auch anorganische Materialien, wie Kaolin oder Bentonit in Frage.

Bei der hautseitigen, den Feuchtigkeitszutritt zur Matrix kontrollierenden Schicht (13, 23) ist auf die Wahl der Dicke und der zu verwendenden Materialien besonderes Gewicht zu legen, da beide Faktoren gemeinsam den zeitlichen Verlauf der feuchtigkeitsbedingten Quellung der Inseln (14, 24) und damit das Ausmaß der Übersättigung wesentlich bestimmen. Erfolgt der Zutritt der Hautfeuchtigkeit zu rasch, so tritt in den Inseln die Übersättigung zu schnell und in zu hohem Maße ein, so daß Ausfällung des Wirkstoffes und somit Zusammenbruch der Freisetzungsrate auf das Sättigungsflußniveau die Folge wäre. Diffundiert die Feuchtigkeit zu langsam in die Matrix, so entsteht der übersättigte Zustand zu spät, und das in den Inseln vorhandene Freisetzungspotential wird nicht optimal genutzt.

Geeignete Grundpolymere für die die Feuchtigkeitseinwanderung kontrollierende Schicht (13, 23) sind zum Beispiel Polyacrylsäureester und Polymethacrylsäureester, Polyvinylalkohol und seine Ester, Polyisobutylen oder Polyethylen. Es ist nicht erforderlich, daß der Hautfeuchtigkeitszutritt durch die Diffusivität des verwendeten Materials kontrolliert wird - ebensogut kann der Zutritt über die Porosität des verwendeten Materials kontrolliert werden. Auch läßt sich der Feuchtigkeitszutritt durch Zusatz von inerten, pulverförmigen Zuschlagstoffen, wie z.B. Talkum, Quarzpulver, Aktivkohle etc. steuern.

Durch gezielte Auswahl des Grundpolymers oder durch hierfür geeignete Zusätze, wie zum Beispiel Harze und Weichmacher läßt sich in einer besonderen Ausführungsform der Erfindung die den Hautfeuchtigkeitszutritt kontrollierende Schicht (13, 23) haftklebend ausführen.

Das Verfahren zur Herstellung solcher Systeme kann in unterschiedlicher Weise ausgeführt werden.

So muß bei der Herstellung der Inseln (14, 24) auf vollständige Lösung des Wirkstoffs im Basismaterial geachtet werden, damit keine Kristallisationskeime in die Arzneiformulierung gebracht werden.

Daher beruhen bevorzugte Verfahren auf gemeinsamer Lösung von Wirkstoff und Hilfsstoffen in hierfür geeigneten Lösungsmitteln und anschließender Trocknung.

Besonders bevorzugt ist hierbei das Verfahren der Sprühtrocknung, bei dem eine Kombination eines Trocknungs- und eines Zerkleinerungsverfahrens vorliegt, so daß die gewünschten Partikel in einem Arbeitsgang erhalten werden. Es ist aber auch möglich, die Lösung von Wirkstoff und Hilfsstoffen auf Metallwalzen oder dehäsiv ausgerüstete Träger auszustreichen, in dieser dünnen Schicht zu trocknen und anschließend mit einem der zahlreichen, dem Fachmann bekannten Verfahren trocken zu vermahlen.

Bei einer weiteren geeigneten Variante wird der Lösung von Wirkstoff und Hilfsstoffen ein Fällungsmittel zugesetzt, das zur partikulären Ausfällung zumindestens eines Teils der Hilfsstoffe führt. Bei diesem Vorgang wird gelöster Wirkstoff in den Basismaterial-Partikeln festgehalten. Nach Abtrennung der Partikel durch Filtration, Sichtung, Naßsiebung oder durch ein sonstiges, diesem Zweck dienendes Verfahren werden die Teilchen getrocknet.

Liegt das Basismaterial vor der Herstellung bereits in geeigneter Korngrößenverteilung vor, so ist es auch möglich, diese Teilchen in einer Lösung des Wirkstoffes zu dispergieren und so durch Absorption eine Sättigung des Basismaterials mit Wirkstoff zu erreichen. Auch hier muß nach einem Trennvorgang wie beispielsweise Filtrieren, Sieben, Sichten u.s.w. ein Trocknungsvorgang (z.B. Hordentrocknung oder Trocknung in der Wirbelschicht) folgen, um die erfindungsgemäßen Inseln (14, 24) zu erzeugen.

Die Korngröße der Inseln sollte stets geringer als die angestrebte Dicke der Matrix (12,22) sein. Deutlich niedrigere Korngrößen sind bevorzugt, da durch die Auftrennung in möglichst viele Einzelkompartimente die Fortpflanzung eventuel ler, während des Tragens des Systems auftretender Kristallisationen wirksam verhindert werden kann.

Aus diesem Grunde werden Partikelgrößen von maximal 5 bis 20 µm, vorzugsweise unter 5 µm bevorzugt.

Der Anteil des Wirkstoffs am Basismaterial der Inseln liegt typischerweise 5 bis 50 % unter der Sättigungslöslichkeit des Wirkstoffes im trockenen Basismaterial. Bei besonders spröden, glasartigen Basismaterialien kann die Sättigungslöslichkeit sogar um bis zu ca. 300 % überschritten werden, da in solchen Grundlagen die Kristallisation mit einer extremen Verzögerung auftritt.

Zur Herstellung der Matrixschicht kann das Grundmaterial in einem Lösungsmittel, in welchem das Basismaterial der Insel unlöslich ist, gelöst, und die Partikel in diesem dispergiert werden. Eine derartige Suspension wird vorteilhaft in einer Streichvorrichtung auf einem dehäsiv ausgerüsteten Träger aufgebracht, auf welcher die Matrixschicht z.B. durch Trocknung im Heißluftstrom verfestigt wird. Für diesen Zweck geeignete Lösungsmittel sind Benzingemische mit geeignetem Siedebereich, Toluol, Methylenchlorid und viele andere lipophile, leichtflüchtige Stoffe, welche die Struktur der hydrophilen Inseln weitgehend unbeeinflußt lassen.

Für ausreichend temperaturstabile Wirkstoffe eignen sich besonders lösungsmittelfreie Verfahren. Beispielsweise kann das Grundmaterial (15) durch Anwendung von Scherkraft und Hitze in einen streichfähigen Zustand gebracht, die erforderliche Menge Inseln eingeknetet oder -gerührt und die Masse nach Aufstreichen auf einen dehäsiven Träger erkalten gelassen werden.

Sowohl das Lösungsmittel- als auch das Schmelzverfahren eignen sich sinngemäß auch für die Herstellung der den Zutritt von Hautfeuchtigkeit kontrollierenden Schicht (13, 23).

In einer besonderen Ausführungsform der Erfindung werden zunächst zwei Schichten Grundmaterial nach einem der vorstehend beschriebenen Verfahren - aber zunächst frei von Inseln - hergestellt.

Beide Schichten bestehen aus dem gleichen Material, sind jedoch nicht notwendigerweise gleich dick. Auf eine der beiden Schichten werden die streufähigen partikulären Inseln homogen (mit stets gleicher Flächenbeladung) aufgestreut. Anschließend wird der zweite Teil der Matrixschicht durch Aufrollen unter Druck aufkaschiert. Erforderlichenfalls kann der Vorgang durch Anwendung von Hitze beschleunigt werden.

Der Mengenanteil des Inselmaterials (Basismaterial und Wirkstoff) an der Matrix ist im wesentlichen abhängig von dem Mengenanteil des Wirkstoffs im Basismaterial der Inseln, der Dicke der Matrixschicht und der erforderlichen Wirkstoffbeladung des transdermalen therapeutischen Systems pro Flächeneinheit. Im allgemeinen wird ein Mengenanteil zwischen 0,5 und 70 %, vorzugsweise zwischen 5 und 40 % angestrebt.

Die Vereinigung von Matrix und der den Zutritt von Hautfeuchtigkeit kontrollierenden Schicht erfolgt zweckmäßig in einer Kaschiereinrichtung, in welcher die beiden Schichten durch Anwendung von Druck zur dauerhaften Adhäsion gebracht werden. Falls erforderlich, kann zur Intensivierung und Beschleunigung des Vorgangs Hitze angewandt werden.

Das fertige System kann mit einer adhäsiven Abdeckung versehen werden. Der Zuschnitt bzw. die Stanzung in beliebige geometrische Formen und Flächengrößen erfolgt in erster Linie nach Maßgabe therapeutischer Richtgrößen, wie z.B. der Zieldosis pro Tag, der Durchlässigkeit der Haut und dem Krümmungsradius des anvisierten Hautareals.

Als Verpackung eignen sich besonders mehrschichtig aufgebaute, als Wasserdampfsperre wirkende Primärverpackungen, beispielsweise aus einem Polyethylencopolymer/Aluminium/Papier-Verbund.

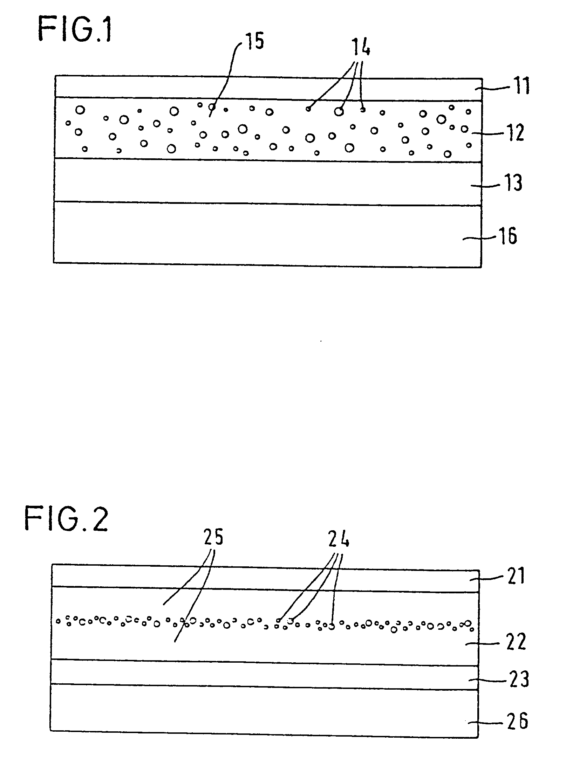

Der Aufbau des transdermalen therapeutischen Systems gemäß der Erfindung ist in den Figuren 1 und 2 dargestellt.

Es bedeuten:

Figur 1:

(11)   Rückschicht
(12)   Matrix mit Inseln (Einschlüssen)
(13)   der Haut zugewandte, den Hautfeuchtigkeitszutritt kontrollierende Schicht
(14)   wirkstoffhaltige Inseln
(15)   Grundmaterial der Matrix
(16)   ablösbare Schutzschicht

Figur 2:

(21)   Rückschicht
(22)   Matrix mit in einer zur Abgabefläche parallelen Anordnung der Inseln
(23)   der Haut zugewandte, den Hautfeuchtigkeitszutritt kontrollierende Schicht
(24)   wirkstoffhaltige Inseln, in einer Ebene angeordnet
(25)   Grundmaterial der Matrix, aus zwei Schichten zusammengefügt
(26)   ablösbare Schutzschicht

Die Erfindung wird anhand der nachfolgenden Beispiele erläutert:

Beispiel 1:

In einem 100-ml-Becherglas mit Magnetrührstab werden 250 mg Haloperidol in 25 g Ethylacetat vollständig gelöst. Daraufhin werden 5 g quervernetztes Polyvinylpyrrolidon (maximale Korngröße 150 Mikrometer) langsam hinzugegeben und die Suspension noch 2 Stunden bei 22 Grad Celsius gerührt. Die gequollenen Teilchen werden zusammen mit dem anhaftenden Lösungsmittel in dünner Schicht auf silikonisierter Polyesterfolie bei 80 Grad Celsius in einem Frischlufttrockenschrank während einer Stunde getrocknet.

4,3 g   der so erhaltenen pulverförmigen Einbettung (enthaltend 205 mg Haloperidol und 4095 mg Polyvinylpyrrolidon)
10,0 g   hitzevulkanisierbares Polydimethylsiloxan und
3,2 g   Benzin

werden unter langsamem Rühren zu einer äußerlich homogenen Masse vereinigt, ohne daß Luftblasen eingearbeitet werden. Die Masse wird mit einem Streichrakel (Spaltbreite 300 Mikrometer) auf einen Träger aus 25 Mikrometer starkem Polyester aufgestrichen und eine halbe Stunde bei einer Temperatur von 80°C vernetzt und gleichzeitig getrocknet. Die auspolymerisierte Schicht wird anschließend wiederum mit einem Streichrakel (Spalt 100 Mikrometer) mit handelsüblicher Silikonklebmasse überzogen, die endgültige Trocknung erfolgt während 30 Minuten offen bei Raumtemperatur und anschließend 10 Minuten bei 50°C im Trockenschrank.

Beispiel 2:

(Vergleichsbeispiel zu Beispiel 1)

0,205 g Haloperidol
4,095 g quervernetztes Polyvinylpyrrolidon
10,0 g hitzevulkanisierbares Polydimethylsiloxan und
3,2 g Benzin

werden unter langsamem Rühren luftblasenfrei zu einer äußerlich homogenen Masse vereinigt. Die Weiterverarbeitung (Streichen, Vernetzen, Überziehen mit Siliconklebmasse, Trocknen) erfolgt wie in Beispiel 1 beschrieben.

Beispiel 3:

Bestimmung der Hautpermeation

Kreisförmige Stanzlinge der Rezepturen gemäß den Beispielen 1 und 2 (Fläche 2,54 cm²) werden auf ein Stück exzidierter Mäusehaut (hairless mice skin) mittig geklebt. Die Hautstücke sind in eine Permeationsapparatur eingespannt, deren prinzipieller Aufbau zum Beispiel bei Kondo et al., J.Pharmacobio.-Dyn. 10, 662-668 (1987) beschrieben ist. Die verwendete Zelle enthielt als Akzeptorlösung Phosphatpuffer eines pH-Wertes von 5,5 und wurde auf 37,0 °C über einen Temperiermantel thermostatisiert.

Die Bestimmung der freigesetzten Haloperidolmenge erfolgte über Hochdruckflüssigkeitschromatographie (reverse phase, Detektion UV bei 242 nm).

Es wurden folgende Messergebnisse erhalten:

| Beispiel | Über 24 Stunden freigesetzte Menge Haloperidol (µg) (auf 2,54 cm$^2$) |
|---|---|
| 1 | 68 |
| 2 | 36 |

Mit dem erfindungsgemäßen Aufbau gemäß Beispiel 1 wird also gegenüber dem Vergleichsbeispiel 2 eine deutlich verstärkte Hautpermeation erzielt.

## Patentansprüche

**Patentansprüche für folgende Vertragsstaaten : AT, BE, CH, DE, DK, FR, GB, IT, LI, LU, NL, SE**

1. Transdermales therapeutisches System mit einem schichtweisen Aufbau aus einer im wesentlichen wirkstoffundurchlässigen Rückschicht (11), einer den Wirkstoff enthaltenden aktivierbaren Matrix (12), die durch Hautfeuchtigkeit aktivierbar ist, dadurch gekennzeichnet, daß es eine den Zutritt von Hautfeuchtigkeit kontrollierende Schicht (13) aufweist und daß die Matrix aus einem wasserdampfdurchlässigen, im wesentlichen wasserunlöslichen und weitgehend wirkstofffreien Grundmaterial (15) besteht, in welchem aus einer festen Lösung eines Arzneiwirkstoffes in einem wasserlöslichen oder in Wasser quellbaren Basismaterial bestehende Inseln (14) verteilt sind.

2. Transdermales therapeutisches System nach Anspruch 1, dadurch gekennzeichnet, daß der Anteil der Inseln (14) an der Masse der Matrixschicht (12) zwischen 0,5 und 70 %, vorzugsweise zwischen 5 und 40 % liegt.

3. Transdermales therapeutisches System nach Anspruch 1 oder 2, dadurch gekennzeichnet, daß die Partikelgröße der Inseln (14) 5- 20 µm, vorzugsweise unter 5 µm beträgt.

4. Transdermales therapeutisches System nach einem oder mehreren der Ansprüche 1 bis 3, dadurch gekennzeichnet, daß die Inseln (14,24) innerhalb des Grundmaterials (15,25) in einer zur Abgabefläche parallelen Ebene angeordnet sind.

5. Transdermales therapeutisches System nach einem oder mehreren der Ansprüche 1 bis 4, dadurch gekennzeichnet, daß das Basismaterial der wasseraufnehmenden Inseln (14, 24) aus Polyvinylalkohol, Polyvinylpyrrolidon, Copolymeren der Polymethacrylsäure, Polysacchariden, Polyethylenglykol oder homogenen Mischungen dieser Stoffe besteht.

6. Transdermales therapeutisches System nach einem oder mehreren der Ansprüche 1 bis 5, dadurch gekennzeichnet, daß in den Inseln (14, 24) ein Wirkstoff aus den Gruppen der Antihistaminika, Antirheumatika, Opioide, Anticholinergika, Antisympathotonika, Steroidhormone, Prostaglandine, Neuroleptika oder Amphetaminderivate Verwendung findet.

7. Transdermales therapeutisches System nach einem oder mehreren der Ansprüche 1 bis 6, dadurch gekennzeichnet, daß die den Zutritt von Hautfeuchtigkeit kontrollierende Schicht (13, 23) haftklebende Eigenschaften besitzt.

**8.** Transdermales therapeutisches System nach einem oder mehreren der Ansprüche 1 bis 7, <u>dadurch gekennzeichnet</u>, daß die den Zutritt von Hautfeuchtigkeit kontrollierende Schicht (13, 23) aus einer Mischung einer oder mehrerer der Komponenten Polyisobutylen, aliphatischen Kohlenwasserstoffharzen, Estern der Polyacrylsäure oder Silikonpolymeren gegebenfalls unter Zusatz geeigneter Additive wie z.B. Weichmachern besteht.

**9.** Transdermales therapeutisches System nach einem oder mehreren der Ansprüche 1 bis 8, <u>dadurch gekennzeichnet</u>, daß die den Zutritt von Hautfeuchtigkeit kontrollierende Schicht (13, 23), die Matrix (12,22) oder beide Schichten einen oder mehreren Zusatzstoffe enthalten, welche die Durchlässigkeit der Haut für den Wirkstoff erhöhen.

**10.** Transdermales therapeutisches System nach einem oder mehreren der Ansprüche 1 bis 9, <u>dadurch gekennzeichnet</u>, daß die den Zutritt von Hautfeuchtigkeit kontrollierende Schicht (13,23) während der Lagerung mit einer ablösbaren Schutzschicht (16, 26) abgedeckt ist.

**11.** Transdermales therapeutisches System nach einem oder mehreren der Ansprüche 1 bis 10, <u>dadurch gekennzeichnet</u>, daß das wasserunlösliche Grundmaterial (15, 25) der Matrix (12, 22) im wesentlichen aus einer oder mehreren der Komponenten Polyisobutylen, natürlichen oder synthetischen Kohlenwasserstoffharzen, Styrol, Butadien-Copolymerisaten, Polyacrylsäureestern, Estern des Polyvinylalkohols oder Silikonpolymeren besteht.

**12.** Verfahren zur Herstellung transdermaler therapeutischer Systeme nach einem oder mehreren der Ansprüche 1 bis 11, <u>dadurch gekennzeichnet</u>, daß die Inseln (14, 24) durch Sprühtrocknung einer Lösung des Wirkstoffes und der feuchtigkeitsabsorbierenden Grundlage in einem geeigneten Lösungsmittel erhalten werden.

**13.** Verfahren zur Herstellung transdermaler therapeutischer Systeme nach einem oder mehreren der Ansprüche 1 bis 11, <u>dadurch gekennzeichnet</u>, daß die Inseln (14, 24) durch Trocknung in dünner Schicht einer Lösung des Wirkstoffs und der feuchtigkeitsabsorbierenden Grundlage in einem hierzu geeigneten Lösungsmittel und anschließende Mahlung erhalten werden.

**14.** Verfahren zur Herstellung transdermaler therapeutischer Systeme nach einem oder mehreren der Ansprüche 1 bis 11, <u>dadurch gekennzeichnet</u>, daß die Inseln (14, 24) durch Fällung aus einer Lösung des Wirkstoffes und der feuchtigkeitsabsorbierenden Grundlage mittels eines hierzu geeigneten Fällungsmittels gebildet werden, in welchem die feuchtigkeitsabsorbierende Grundlage im wesentlichen unlöslich ist.

**15.** Verfahren zur Herstellung transdermaler therapeutischer Systeme nach einem oder mehreren der Ansprüche 1 bis 11, <u>dadurch gekennzeichnet</u>, daß die Beladung der Inseln (14, 24) mit Wirkstoff in einer Suspension unbeladener Inseln in einem geeigneten, den Wirkstoff gelöst enthaltenden Lösungsmittel durch Fest/Flüssig-Absorption erfolgt.

**16.** Verfahren zur Herstellung transdermaler therapeutischer Systeme nach einem oder mehreren der Ansprüche 1 bis 11, <u>dadurch gekennzeichnet</u>, daß die Matrix (12, 22) oder die den Zutritt von Hautfeuchtigkeit kontrollierende Schicht (13, 23) in einer Lösung oder Suspension in einem geeigneten Lösungsmittel auf einen dehäsiv ausgerüsteten Träger aufgebracht werden und das Lösungsmittel anschließend durch Trocknung entfernt wird.

**17.** Verfahren zur Herstellung transdermaler therapeutischer Systeme nach einem oder mehreren der Ansprüche 1 bis 11, <u>dadurch gekennzeichnet</u>, daß die Matrix (12, 22) oder die den Zutritt von Hautfeuchtigkeit kontrollierende Schicht (13, 23) durch Hitzeanwendung verflüssigt und in diesem Zustand auf einen dehäsiv ausgerüsteten Träger aufgebracht wird.

**18.** Verfahren zur Herstellung transdermaler therapeutischer Systeme nach einem oder mehreren der Ansprüche 1 bis 11, <u>dadurch gekennzeichnet</u>, daß die Matrix (12, 22), die den Zutritt von Hautfeuchtigkeit kontrollierende Schicht (13, 23) und die Rückschicht (11, 21) durch Druck- und/oder Temperaturanwendung miteinander dauerhaft verbunden werden.

**19.** Verfahren zur Herstellung transdermaler therapeutischer Systeme nach einem oder mehreren der Ansprüche 1 bis 11, <u>dadurch gekennzeichnet</u>, daß die Matrix (22) durch getrenntes Herstellen zweier gleichartiger Schichten Grundmaterial (25) mit gegebenenfalls unterschiedlicher Schichtdicke, Aufstreuen der Inseln (24) auf eine der beiden Schichten und anschließendes Aufbringen der zweiten Schicht des Grundmaterials (25) unter Anwendung von Druck und/oder Temperatur gebildet wird.

**Patentansprüche für folgende Vertragsstaaten : ES, GR**

1. Verfahren zur Herstellung eines transdermalen therapeutischen Systems mit einem schichtweisen Aufbau aus einer im wesentlichen wirkstoffundurchlässigen Rückschicht (11), einer den Wirkstoff enthaltenden aktivierbaren Matrix (12) und einer den Zutritt von Hautfeuchtigkeit kontrollierenden Schicht (13), bei welchem Verfahren eine Matrix, die durch Hautfeuchtigkeit aktivierbar ist, aus einem wasserdampfdurchlässigen, im wesentlichen wasserunlöslichen und weitgehend wirkstofffreien Grundmaterial (15) verwendet wird, in welchem aus einer festen Lösung eines Arzneiwirkstoffes in einem wasserlöslichen oder in Wasser quellbaren Basismaterial bestehende Inseln (14) verteilt werden.

2. Verfahren zur Herstellung eines transdermalen therapeutischen Systems nach Anspruch 1, dadurch gekennzeichnet, daß der Anteil der Inseln (14) an der Masse der Matrixschicht (12) zwischen 0,5 und 70 %, vorzugsweise zwischen 5 und 40 % liegen und daß die Partikelgröße der Inseln (14) 5- 20 μm, vorzugsweise unter 5 μm beträgt.

3. Verfahren zur Herstellung eines transdermalen therapeutischen Systems nach Anspruch 1, dadurch gekennzeichnet, daß die Inseln (14,24) innerhalb des Grundmaterials (15,25) in einer zur Abgabefläche parallelen Ebene angeordnet werden.

4. Verfahren zur Herstellung eines transdermalen therapeutischen Systems nach Anspruch 1, dadurch gekennzeichnet, daß ein Basismaterial der wasseraufnehmenden Inseln (14, 24) aus Polyvinylalkohol, Polyvinylpyrrolidon, Copolymeren der Polymethacrylsäure, Polysacchariden, Polyethylenglykol oder homogenen Mischungen dieser Stoffe verwendet wird.

5. Verfahren zur Herstellung eines transdermalen therapeutischen Systems nach Anspruch 1, dadurch gekennzeichnet, daß in den Inseln (14, 24) ein Wirkstoff aus den Gruppen der Antihistaminika, Antirheumatika, Opioide, Anticholinergika, Antisympathotonika, Steroidhormone, Prostaglandine, Neuroleptika oder Amphetaminderivate Verwendung findet.

6. Verfahren zur Herstellung eines transdermalen therapeutischen Systems nach Anspruch 1, dadurch gekennzeichnet, daß die den Zutritt von Hautfeuchtigkeit kontrollierende Schicht (13, 23) haftklebende Eigenschaften besitzt.

7. Verfahren zur Hestellung eines transdermalen therapeutischen Systems nach Anspruch 1, dadurch gekennzeichnet, daß die den Zutritt von Hautfeuchtigkeit kontrollierende Schicht (13, 23) aus einer Mischung einer oder mehrerer der Komponenten Polyisobutylen, aliphatischen Kohlenwasserstoffharzen, Estern der Polyacrylsäure oder Silikonpolymeren gegebenenfalls unter Zusatz geeigneter Additive wie z.B. Weichmachern gebildet wird.

8. Verfahren zur Herstellung eines transdermalen therapeutischen Systems nach Anspruch 1, dadurch gekennzeichnet, daß in die den Zutritt von Hautfeuchtigkeit kontrollierende Schicht (13, 23), die Matrix (12,22) oder beide Schichten eine oder mehrere Zusatzstoffe eingebracht werden, welche die Durchlässigkeit der Haut für den Wirkstoff erhöhen.

9. Verfahren zur Herstellung eines transdermalen therapeutischen Systems nach Anspruch 1, dadurch gekennzeichnet, daß die den Zutritt von Hautfeuchtigkeit kontrollierende Schicht (13,23) während der Lagerung mit einer ablösbaren Schutzschicht (16, 26) abgedeckt ist wird.

10. Verfahren zur Herstellung eines transdermalen therapeutischen Systems nach Anspruch 1, dadurch gekennzeichnet, daß das wasserunlösliche Grundmaterial (15, 25) der Matrix (12, 22) im wesentlichen aus einer oder mehreren der Komponenten Polyisobutylen, natürlichen oder synthetischen Kohlenwasserstoffharzen, Styrol, Butadien-Copolymerisaten, Polyacrylsäureestern, Estern des Polyvinylalkohols oder Silikonpolymeren gebildet wird.

11. Verfahren zur Herstellung eines transdermalen therapeutischen Systems nach Anspruch 1, dadurch gekennzeichnet, daß die Inseln (14, 24) durch Sprühtrocknung einer Lösung des Wirkstoffes und der feuchtigkeitsabsorbierenden Grundlage in einem geeigneten Lösungsmittel erhalten werden.

12. Verfahren zur Herstellung eines transdermalen therapeutischen Systems nach Anspruch 1, dadurch gekennzeichnet, daß die Inseln (14, 24) durch Trocknung in dünner Schicht einer Lösung des Wirkstoffs und der feuchtigkeitsabsorbierenden Grundlage in einem hierzu geeigneten Lösungsmittel und anschließende Mahlung erhalten werden.

13. Verfahren zur Herstellung eines transdermalen therapeutischen Systems nach Anspruch 1, dadurch gekennzeichnet, daß die Inseln (14, 24) durch Fällung aus einer Lösung des Wirkstoffes und der feuchtigkeitsabsorbierenden Grundlage mittels eines hierzu geeigneten Fällungsmittels gebildet werden, in welchem die feuchtigkeitsabsorbierende Grundlage im wesentlichen unlöslich ist.

14. Verfahren zur Herstellung eines transdermalen therapeutischen Systems nach Anspruch 1, dadurch gekennzeichnet, daß die Beladung der Inseln (14, 24) mit Wirkstoff in einer Suspension unbeladener Inseln in einem geeigneten, den Wirkstoff gelöst enthaltenden Lösungsmittel durch Fest/Flüssig-Absorption erfolgt.

15. Verfahren zur Herstellung eines transdermalen therapeutischen Systems nach Anspruch 1, dadurch gekennzeichnet, daß die Matrix (12, 22) oder die den Zutritt von Hautfeuchtigkeit kontrollierende Schicht (13, 23) in einer Lösung oder Suspension in einem geeigneten Lösungsmittel auf einem dehäsiv ausgerüsteten Träger aufgebracht werden und das Lösungsmittel anschließend durch Trocknung entfernt wird.

16. Verfahren zur Herstellung eines transdermalen therapeutischen Systems nach Anspruch 1, dadurch gekennzeichnet, daß die Matrix (12, 22) oder die den Zutritt von Hautfeuchtigkeit kontrollierende Schicht (13, 23) durch Hitzeanwendung verflüssigt und in diesem Zustand auf einen dehäsiv ausgerüsteten Träger aufgebracht wird.

17. Verfahren zur Herstellung eines transdermalen therapeutischen Systems nach Anspruch 1, dadurch gekennzeichnet, daß die Matrix (12, 22), die den Zutritt von Hautfeuchtigkeit kontrollierende Schicht (13, 23) und die Rückschicht (11, 21) durch Druck- und/oder Temperaturanwendung miteinander dauerhaft verbunden werden.

18. Verfahren zur Herstellung eines transdermalen therapeutischen Systems nach Anspruch 1, dadurch gekennzeichnet, daß die Matrix (22) durch getrenntes Herstellen zweier gleichartiger Schichten Grundmaterial (25) mit gegebenenfalls unterschiedlicher Schichtdicke, Aufstreuen der Inseln (24) auf eine der beiden Schichten und anschließendes Aufbringen der zweiten Schicht des Grundmaterials (25) unter Anwendung von Druck und/oder Temperatur gebildet wird.

## Claims

**Claims for the following Contracting States : AT, BE, CH, DE, DK, FR, GB, IT, LI, LU, NL, SE**

1. A transdermal therapeutic system exhibiting a layered structure, comprising a backing layer (11) which is substantially impermeable to active substances, and a matrix (12) containing the active substance and being activatable by cutaneous moisture, characterized in that it is provided with a layer (13) controlling the access of cutaneous moisture, and in that the matrix comprises a basis material (15) which is permeable to water vapour, but substantially water-insoluble and mainly free of active substances, in which islands (14) are distributed which consist of a solid solution of drug in a water-soluble or water-swellable basis material.

2. The transdermal therapeutic system according to claim 1, characterized in that the portion of islands (14) to the mass of the matrix layer (12) lies between 0.5 and 70%, preferably between 5 and 40%.

3. The transdermal therapeutic system according to claims 1 or 2, characterized in that the particle size of the islands (14) amounts to 5 to 20 μm, preferably is below 5 μm

4. The transdermal therapeutic system according to one or more of claims 1 to 3, characterized in that the

9

islands (14, 24) within the basis material (15, 25) are positioned in a plane parallel to the releasing surface.

5. The transdermal therapeutic system according to one or more of claims 1 to 4, characterized in that the basis material of the water-absorbing islands (14, 24) comprises polyvinyl alcohol, polyvinyl pyrrolidone, copolymers of polymethacrylic acid, polysaccharides, polyethylene glycol, or homogeneous mixtures of these substances.

6. The transdermal therapeutic system according to one or more of claims 1 to 5, characterized in that an active substance selected from the group consisting of antihistamines, antirheumatics, opioids, anticholinergics, antisympathotonics, steroid hormones, prostaglandins, neuroleptics, or amphetamine derivatives is used in the islands (14, 24).

7. The transdermal therapeutic system according to one or more of claims 1 to 6, characterized in that the cutaneous moisture access controlling layer (13, 23) exhibits pressure-sensitive adhesive properties.

8. The transdermal therapeutic system according to one or more of claims 1 to 7, characterized in that the cutaneous moisture access controlling layer (13, 23) comprises a mixture of one or more of the components polyisobutylene, aliphatic hydrocarbon resins, esters of polyacrylic acid, or silicone polymers, optionally with the addition of suitable additives, e.g., plasticizers.

9. The transdermal therapeutic system according to one or more of claims 1 to 8, characterized in that the cutaneous moisture access controlling layer (13, 23), the matrix (12, 22), or both of them comprise one or more additives which increase the permeability of the skin to the active substance.

10. The transdermal therapeutic system according to one or more of claims 1 to 9, characterized in that the layer (13, 23) controlling the access of cutaneous moisture is covered with a removable protective layer (16, 26) during storage.

11. The transdermal therapeutic system according to one or more of claims 1 to 10, characterized in that the water-insoluble basis material (15, 25) of matrix (12, 22) substantially comprises one or more of the components polyisobutylene, natural or synthetic hydrocarbon resins, styrene-butadiene copolymers, polyacrylic acid esters, esters of polyvinyl alcohol, or silicone polymers.

12. A process for the production of transdermal therapeutic systems as defined in one or more of claims 1 to 11, characterized in that the islands (14, 24) are obtained by spray drying a solution of the active substance and the moisture absorbing basis material in a suitable solvent.

13. A process for the production of transdermal therapeutic systems as defined in one or more of claims 1 to 11, characterized in that the islands (14, 24) are obtained by drying as thin layer a solution of the active substance and the moisture absorbing basis material in a suitable solvent, and by subsequent milling.

14. A process for the production of transdermal therapeutic systems as defined in one or more of claims 1 to 11, characterized in that the islands (14, 24) are formed by precipitation from a solution of the active substance and the moisture absorbing basis material by means of a suitable precipitation agent in which the moisture absorbing basis material is substantially insoluble.

15. A process for the production of transdermal therapeutic systems as defined in one or more of claims 1 to 11, characterized in that loading the islands (14, 24) with active substance is carried out by means of solid-liquid absorption in a suspension of unloaded islands in a suitable solvent comprising the active substance in dissolved form.

16. A process for the production of transdermal therapeutic systems as defined in one or more of claims 1 to 11, characterized in that the matrix (12, 22) or the layer controlling the access of cutaneous moisture (13, 23) are applied to a carrier, which has been rendered dehesive, in a solution or suspension in a suitable solvent, and that the solvent is subsequently removed by drying.

17. A process for the production of transdermal therapeutic systems as defined in one or more of claims 1 to 11, characterized in that the matrix (12, 22) or the cutaneous moisture access controlling layer (13, 23) is liquefied by application of heat, and applied in this state to a dehesive carrier.

18. A process for the production of transdermal therapeutic systems as defined in one or more of claims 1 to 11, characterized in that the matrix (12, 22), the cutaneous moisture access controlling layer (13, 23), and the backing layer (11, 21) are continuously combined by application of pressure and/or temperature.

19. A process for the production of transdermal therapeutic systems as defined in one or more of claims 1 to 11, characterized in that the matrix (22) is formed by separate manufacture of two identical layers of basis material (25), optionally having a different layer thickness, sprinkling the islands (24) on one of the two layers, and subsequent application of the second layer of basis material (25) under application of pressure and/or temperature.

**Claims for the following Contracting States : ES, GR**

1. A process for the production of a transdermal therapeutic system exhibiting a layered structure, comprising a backing layer (11) which is substantially impermeable to active substances, an activatable matrix (12) containing the active substance and a layer (13) controlling the access of cutaneous moisture, in which process a matrix which is activatable by cutaneous moisture and comprises a basis material (15) which is permeable to water vapour, but substantially water-insoluble and mainly free of active substances, in which islands (14) are distributed which consist of a solid solution of drug in a water-soluble or water-swellable basis material.

2. The process for the production of the transdermal therapeutic system as defined in claim 1, characterized in that the portion of islands (14) to the mass of the matrix layer (12) lies between 0.5 and 70%, preferably between 5 and 40%, and in that the particle size of the islands (14) amounts to 5 to 20 $\mu$m and is preferably below 5 $\mu$m.

3. The process for the production of the transdermal therapeutic system as defined in claim 1, characterized in that the islands (14, 24) within the basis material (15, 25) are positioned in a plane parallel to the releasing surface.

4. The process for the production of the transdermal therapeutic system as defined in claim 1, characterized in that a basis material of the water-absorbing islands (14, 24) comprising polyvinyl alcohol, polyvinyl pyrrolidone, copolymers of polymethacrylic acid, polysaccharides, polyethylene glycol, or homogeneous mixtures of these substances is used.

5. The process for the production of the transdermal therapeutic system as defined in claim 1, characterized in that an active substance selected from the group consisting of antihistamines, antirheumatics, opioids, anticholinergics, antisympathotonics, steroid hormones, prostaglandins, neuroleptics, or amphetamine derivatives is used in the islands (14, 24).

6. The process for the production of the transdermal therapeutic system as defined in claim 1, characterized in that the cutaneous moisture access controlling layer (13, 23) exhibits pressure-sensitive adhesive properties.

7. The process for the production of the transdermal therapeutic system as defined in claim 1, characterized in that the cutaneous moisture access controlling layer (13, 23) is made up of a mixture of one or more of the components polyisobutylene, aliphatic hydrocarbon resins, esters of polyacrylic acid, or silicone polymers, optionally with the addition of suitable additives, e.g., plasticizers.

8. The process for the production of the transdermal therapeutic system as defined in claim 1, characterized in that one or more additives which increase the permeability of the skin to the active substance are introduced into the cutaneous moisture access controlling layer (13, 23), the matrix (12, 22), or both of them.

9. The process for the production of the transdermal therapeutic system as defined in claim 1, characterized in that the layer (13, 23) controlling the access of cutaneous moisture is covered with a removable protective layer (16, 26) during storage.

10. The process for the production of the transdermal therapeutic system as defined in claim 1, characterized in that the water-insoluble basis material (15, 25) of matrix (12, 22) is substantially made up of one or more of the components polyisobutylene, natural or synthetic hydrocarbon resins, styrene-butadiene copolymers, polyacrylic acid esters, esters of polyvinyl alcohol, or silicone polymers.

11. The process for the production of the transdermal therapeutic system as defined in claim 1, characterized in that the islands (14, 24) are obtained by spray drying a solution of the active substance and the moisture absorbing basis material in a suitable solvent.

12. The process for the production of the transdermal therapeutic system as defined in claim 1, characterized in that the islands (14, 24) are obtained by drying as thin layer a solution of the active substance and the moisture absorbing basis material in a suitable solvent, and by subsequent milling.

13. The process for the production of the transdermal therapeutic system as defined in claim 1, characterized in that the islands (14, 24) are formed by precipitation from a solution of the active substance and the moisture absorbing basis material by means of a suitable precipitation agent in which the moisture absorbing basis material is substantially insoluble.

14. The process for the production of the transdermal therapeutic system as defined in claim 1, characterized in that loading the islands (14, 24) with active substance is carried out by means of solid-liquid absorption in a suspension of unloaded islands in a suitable solvent comprising the active substance in dissolved form.

15. The process for the production of the transdermal therapeutic system as defined in claim 1, characterized in that the matrix (12, 22) or the layer controlling the access of cutaneous moisture (13, 23) are applied to a carrier, which has been rendered dehesive, in a solution or suspension in a suitable solvent, and that the solvent is subsequently removed by drying.

16. The process for the production of the transdermal therapeutic system as defined in claim 1, characterized in that the matrix (12, 22) or the cutaneous moisture access controlling layer (13, 23) is liquefied by application of heat, and applied in this state to a dehesive carrier.

17. The process for the production of the transdermal therapeutic system as defined in claim 1, characterized in that the matrix (12, 22), the cutaneous moisture access controlling layer (13, 23), and the backing layer (11, 21) are continuously combined by application of pressure and/or temperature.

18. The process for the production of the transdermal therapeutic system as defined in claim 1, characterized in that the matrix (22) is formed by separate manufacture of two identical layers of basis material (25), optionally having a different layer thickness, sprinkling the islands (24) on one of the two layers, and subsequent application of the second layer of basis material (25) under application of pressure and/or temperature.

## Revendications

**Revendications pour les Etats contractants suivants : AT, BE, CH, DE, DK, FR, GB, IT, LI, LU, NL, SE**

1. Système thérapeutique, transdermique, multicouche, constitué d'une couche dorsale (11) essentiellement imperméable au principe actif, d'une matrice (12) activable, contenant le principe actif, qui est activable par l'humidité cutanée, caractérisé en ce qu'il présente une couche commandant la pénétration de l'humidité cutanée et en ce que la matrice est constituée par une matière de base (15) perméable à la vapeur d'eau, essentiellement insoluble dans l'eau et en grande partie dépourvue de principe actif, dans laquelle sont répartis ou distribués des ilôts (14) constitués d'une solution solide d'un principe médicalement actif dans une matière de base soluble dans l'eau ou gonflable dans l'eau.

2. Système thérapeutique, transdermique, suivant la revendication 1, caractérisé en ce que la proportion des ilôts (14) par rapport à la masse de la couche constituant la matrice (12) fluctue de 0,5 à 70%, de préférence de 5 à 40%.

3. Système thérapeutique, transdermique, suivant la revendication 1 ou 2, caractérisé en ce que le calibre des particules des ilôts (14) varie de 5 à 20 $\mu$m et est de préférence inférieur à 5 $\mu$m.

**4.** Système thérapeutique, transdermique, suivant une ou plusieurs des revendications 1 à 3, caractérisé en ce que les îlôts (14, 24) sont agencés en un plan parallèle à la surface administrante à l'intérieur de la matière de base (15, 25).

**5.** Système thérapeutique, transdermique, suivant une ou plusieurs des revendications 1 à 4, caractérisé en ce que la matière de base des îlôts (14, 24) absorbant l'eau, est constituée de poly(alcool vinylique), de polyvinylpyrrolidone, de copolymères du poly(acide méthacrylique), de polysaccharides, de polyéthylèneglycol ou de mélanges homogènes de ces matières.

**6.** Système thérapeutique, transdermique, suivant une ou plusieurs des revendications 1 à 5, caractérisé en ce que l'on utilise dans les îlôts (14, 24) un principe actif appartenant aux groupes des antihistaminiques, des antirhumatismaux, des opioïdés, des anticholinergiques, des antisympathicotoniques, des hormones stéroïdiennes, des prostaglandines, des neuroleptiques, ou des dérivés de l'amphétamine.

**7.** Système thérapeutique, transdermique, suivant une ou plusieurs des revendications 1 à 6, caractérisé en ce que la couche commandant la pénétration de l'humidité cutanée (13, 23) possède des propriétés autoadhésives.

**8.** Système thérapeutique, transdermique, suivant une ou plusieurs des revendications 1 à 7, caractérisé en ce que la couche (13, 23) commandant la pénétration de l'humidité cutanée, est constituée d'un mélange d'un ou plusieurs des composants suivants : polyisobutylène, résines d'hydrocarbures aliphatiques, esters du poly(acide acrylique) ou polymères de silicones, éventuellement sous addition d'additifs convenables, comme, par exemple des plastifiants.

**9.** Système thérapeutique, transdermique, suivant une ou plusieurs des revendications 1 à 8, caractérisé en ce que la couche (13, 23) commandant la pénétration de l'humidité cutanée, la matrice (12, 22) ou les deux couches contiennent un ou plusieurs additifs qui augmentent la perméabilité de la peau au principe actif.

**10.** Système thérapeutique, transdermique, suivant une ou plusieurs des revendications 1 à 9, caractérisé en ce que la couche (13, 23) commandant la pénétration de l'humidité cutanée est recouverte, au cours de la conservation, d'une couche protectrice détachable ou amovible (16, 26).

**11.** Système thérapeutique, transdermique, suivant une ou plusieurs des revendications 1 à 10, caractérisé en ce que la matière de base (15, 25) insoluble dans l'eau de la matrice (12, 22) est essentiellement constituée d'un ou plusieurs des composants qui suivent : polyisobutylène, résines d'hydrocarbures naturelles ou synthétiques, styrène, copolymères du butadiène, esters du poly(acide acrylique), esters du poly(alcool vinylique) ou polymères de silicones.

**12.** Procédé de préparation de systèmes thérapeutiques, transdermiques, suivant une ou plusieurs des revendications 1 à 11, caractérisé en ce que l'on obtient les îlôts (14, 24) par séchage par pulvérisation d'une solution du principe actif et de la matière de base absorbant l'humidité dans un solvant convenable.

**13.** Procédé de préparation de systèmes thérapeutiques, transdermiques, suivant une ou plusieurs des revendications 1 à 11, caractérisé en ce que les îlôts (14, 24) sont obtenus par séchage en couche mince d'une solution du principe actif et de la matière de base absorbant 'humidité dans un solvant approprié à cette fin et broyage subséquent.

**14.** Procédé de préparation de systèmes thérapeutiques, transdermiques, suivant une ou plusieurs des revendications 1 à 11, caractérisé en ce que les îlôts (14, 24) sont formés par précipitation à partir d'une solution du principe actif et de la matière de base absorbant l'humidité à l'aide d'un agent de précipitation approprié à cette fin, dans lequel la matière de base absorbant l'humidité est essentiellement insoluble.

**15.** Procédé de préparation de systèmes thérapeutiques, transdermiques, suivant une ou plusieurs des revendications 1 à 11, caractérisé en ce que le chargement des îlôts (14, 24) par le principe actif se réalise dans une suspension d'îlôts non chargés dans un solvant approprié, contenant le principe actif en solution, par absorption solide/liquide.

**16.** Procédé de préparation de systèmes thérapeutiques, transdermiques, suivant une ou plusieurs des revendications 1 à 11, caractérisé en ce que la matrice (12, 22), ou la couche (13, 23) commandant la pé-

13

nétration de l'humidité cutanée, est appliquée dans une solution ou une suspension dans un solvant convenable sur un support rendu antiadhésif et le solvant est ensuite éliminé par séchage.

17. Procédé de préparation de systèmes thérapeutiques, transdermiques, suivant une ou plusieurs des revendications 1 à 11, caractérisé en ce que la matrice (12, 22), ou la couche (13, 23) commandant la pénétration de l'humidité cutanée est liquéfiée par l'utilisation de chaleur et appliquée dans cet état sur un support rendu antiadhésif.

18. Procédé de préparation de systèmes thérapeutiques, transdermiques, suivant une ou plusieurs des revendications 1 à 11, caractérisé en ce que la matrice (12, 22), la couche (13, 23) commandant la pénétration de l'humidité cutanée et la couche dorsale (11, 21) sont mutuellement liées de façon permenante par l'utilisation de pression et/ou de températures.

19. Procédé de préparation de systèmes thérapeutiques, transdermiques, suivant une ou plusieurs des revendications 1 à 11, caractérisé en ce que la matrice (22) est formée par préparation séparée de deux couches identiques de matière de base (25) mais d'une épaisseur de couche éventuellement différente, saupoudrage des îlots (24) sur l'une des deux couches et application subséquente de la seconde couche de la matière de base (25) en recourant à l'utilisation de pression et/ou de températures.

**Revendications pour les Etats contractants suivants : ES, GR**

1. Procédé de préparation d'un système thérapeutique, transdermique, multicouche, constitué d'une couche dorsale (11) essentiellement imperméable au principe actif, d'une matrice activable (12) contenant le principe actif et d'une couche (13) commandant la pénétration de l'humidité cutanée, conformément auquel on utilise une matrice, qui est activable par l'humidité cutanée en une matière de base (15) perméable à la vapeur d'eau, essentiellement imperméable à l'eau et en grande partie dépourvue de principe actif, dans laquelle sont répartis des îlots (14) constitués d'une solution solide d'un principe actif médicamenteux dans une matière de base soluble dans l'eau ou gonflable dans l'eau.

2. Procédé de préparation d'un système thérapeutique, transdermique, suivant la revendication 1, caractérisé en ce que la proportion des îlots (14) par rapport à la masse de la couche constituant la matrice (12) fluctue de 0,5 à 70%, de préférence de 5 à 40% et en ce que le calibre des particules des îlots (14) varie de 5 à 20 $\mu$m et est de préférence inférieur à 5 $\mu$m.

3. Procédé de préparation d'un système thérapeutique, transdermique, suivant la revendication 1, caractérisé en ce que les îlots (14, 24) sont agencés en un plan parallèle à la surface administrante à l'intérieur de la matière de base (15, 25).

4. Procédé de préparation d'un système thérapeutique, transdermique, suivant la revendication 1, caractérisé en ce qu'on utilise une matière de base des îlots absorbant l'eau (14, 24) constituée de poly(alcool vinylique) de polyvinylpyrrolidone, de copolymères du poly(acide méthacrylique), de polysaccharides, de polyéthylèneglycol ou de mélanges homogènes de ces matières.

5. Procédé de préparation d'un système thérapeutique, transdermique, suivant la revendication 1, caractérisé en ce que l'on utilise dans les îlots (14, 24) un principe actif appartenant aux groupes des anthistaminiques, des antirhumatismaux, des opioïdés, des anticholinergiques, des antisympathicotoniques, des hormones stéroïdiennes, des prostaglandines, des neuroleptiques, ou des dérivés de l'amphétamine.

6. Procédé de préparation d'un système thérapeutique, transdermique, suivant la revendication 1, caractérisé en ce que la couche commandant la pénétration de l'humidité cutanée (13, 23) possède des propriétés autoadhésives.

7. Procédé de préparation d'un système thérapeutique, transdermique, suivant la revendication 1, caractérisé en ce que la couche (13, 23) commandant la pénétration de l'humidité cutanée est formée à partir d'un mélange d'un ou plusieurs des composants qui suivent : polyisobutylène, résines d'hydrocarbures aliphatiques, esters du poly(acide acrylique) ou polymères de silicones, éventuellement sous addition d'additifs appropriés, comme, par exemple des plastifiants.

8. Procédé de préparation d'un système thérapeutique, transdermique, suivant la revendication 1, caractérisé en ce que dans la couche (13, 23) commandant la pénétration de l'humidité cutanée, la matrice (12, 22), ou ces deux couches, on introduit un ou plusieurs additifs qui augmentent la perméabilité de la peau au principe actif.

9. Procédé de préparation d'un système thérapeutique, transdermique, suivant la revendication 1, caractérisé en en que, que cours de la conservation, on recouvre la couche (13, 23) commandant la pénétration de l'humidité cutanée d'une couche protectrice, détachable ou amovible (16, 26).

10. Procédé de préparation d'un système thérapeutique, transdermique, suivant la revendication 1, caractérisé en ce que la matière de base (15, 25) insoluble dans l'eau de la matrice (12, 22) est essentiellement formée à partir d'un ou plusieurs des composants qui suivent : polyisobutylène, résines d'hydrocarbures naturelles ou synthétiques, styrène, copolymères de butadiène, esters du poly(acide acrylique), esters du poly(alcool vinylique) ou polymères de silicones.

11. Procédé de préparation d'un système thérapeutique, transdermique, suivant la revendication 1, caractérisé en ce que l'on obtient les îlots (14, 24) par séchage par pulvérisation d'une solution du principe actif et de la matière de base absorbant l'humidité dans un solvant convenable.

12. Procédé de préparation d'un système thérapeutique, transdermique, suivant la revendication 1, caractérisé en ce que les îlots (14, 24) sont obtenus par séchage en couche mince d'une solution du principe actif et de la matière de base absorbant l'humidité dans un solvant approprié à cette fin et broyage subséquent.

13. Procédé de préparation d'un système thérapeutique, transdermique, suivant la revendication 1, caractérisé en ce que les îlots (14, 24) sont formés par précipitation à partir d'une solution du principe actif et de la matière de base absorbant l'humidité à l'aide d'un agent de précipitation approprié à cette fin, dans lequel la matière de base absorbant l'humidité est essentiellement insoluble.

14. Procédé de préparation d'un système thérapeutique, transdermique, suivant la revendication 1, caractérisé en ce que le chargement des îlots (14, 24) par le principe actif se réalise dans une suspension d'îlots non chargés dans un solvant approprié, contenant le principe actif en solution, par absorption solide/liquide.

15. Procédé de préparation d'un système thérapeutique, transdermique, suivant la revendication 1, caractérisé en ce que la matrice (12, 22), ou la couche (13, 23) commandant la pénétration de l'humidité cutanée, est appliquée dans une solution ou une suspension dans un solvant convenable sur un support rendu antiadhésif et le solvant est ensuite éliminé par séchage.

16. Procédé de préparation d'un système thérapeutique, transdermique, suivant la revendication 1, caractérisé en ce que la matrice (12, 22) ou la couche (13, 23) commandant la pénétration de l'humidité cutanée est liquéfiée par l'utilisation de chaleur et appliquée dans cet état sur un support rendu antiadhésif.

17. Procédé de préparation d'un système thérapeutique, transdermique, suivant la revendication 1, caractérisé en ce que la matrice (12, 22) la couche (13, 23) commandant la pénétration de l'humidité cutanée et la couche dorsale (11, 21) sont mutuellement liées de façon permanente par l'utilisation de pression et/ou de températures.

18. Procédé de préparation d'un système thérapeutique, transdermique, suivant la revendication 1, caractérisé en ce que la matrice (22) est formée par préparation séparée de deux couches identiques de matière de base (25) mais d'une épaisseur de couche éventuellement différente, saupoudrage des îlots (24) sur l'une des deux couches et application subséquente de la seconde couche de la matière de base (25) en recourant à l'utilisation de pression et/ou de températures.

# FIG.1

15    14    11
      12
      13
      16

# FIG.2

25    24    21
      22
      23
      26